# EUROPEAN PATENT APPLICATION

(11) **EP 3 698 804 A2**
(43) Date of publication of application: **26.08.2020**
(21) Application number: 18868101.9
(22) Date of filing: 11.10.2018
(51) Int. Cl.: A61K 36/23, A61K 31/4365, A61K 31/4709

(54) **ANTITHROMBOTIC COMPOSITION CONTAINING WILD PARSLEY EXTRACT AND ANTITHROMBOTIC AGENT**

(30) Priority: 18.10.2017 KR 20170135472
(71) Applicant: Korean Drug Co., Ltd., Icheon-si, Gyeonggi-do 17303 (KR)
(72) Inventor: PARK, Jeehun, Seoul 08861 (KR); CHOI, Jungho, Daejeon 34049 (KR); KIM, Riyeon, Suwon-si Gyeonggi-do 16713 (KR); YU, Juhee, Gyeongsan-si Gyeongsangbuk-do 38686 (KR)
(74) Representative: Maiwald Patent- und Rechtsanwaltsgesellschaft mbH
(86) International application number: PCT/KR2018/011971
(87) International publication number: WO 2019/078543

(57) **Abstract**

The present invention relates to a composition comprising Oenanthe javanica extract and one or more kinds selected from the group consisting of Clopidogrel or its pharmaceutically acceptable salt and Cilostazol or its pharmaceutically acceptable salt as an antithrombotic agent, and a method for inhibiting thrombi or for preventing or treating thrombus-related diseases using the same, and a method for preparing thereof, and more specifically, it relates to a pharmaceutical composition for antithrombosis, or for preventing or treating thrombus-related diseases, which comprises Oenanthe javanica extract and one or more kinds selected from the group consisting of Clopidogrel or its pharmaceutically acceptable salt and Cilostazol or its pharmaceutically acceptable salt as active ingredients, and exhibits an excellent inhibitory action of thrombosis when administered alone, compared to combinations of other antithrombotic agents.

## Description

### [TECHNICAL FIELD]

### Cross-reference with related application(s)

The present application claims the benefit of priority based on Korean Patent Application No. 10-2017-0135472 filed on October 18, 2017, and the entire contents disclosed in the corresponding Korean patent application are incorporated as part of the present specification.

The present invention relates to a composition comprising Oenanthe javanica extract and an antithrombotic agent, a method for inhibiting thrombi, or a method for prevention or treatment of thrombus-related diseases, which is characterized by administering the same, and a method for preparation of the pharmaceutical composition, and more specifically, it relates to a pharmaceutical composition for antithrombosis , or for preventing or treating thrombus-related diseases, comprising Oenanthe javanica extract, and one or more kinds selected from the group consisting of Clopidogrel or its pharmaceutically acceptable salt and Cilostazol or its pharmaceutically acceptable salt as an antithrombotic agent, as active ingredients, a method for inhibiting thrombi, or a method for prevention or treatment of thrombus-related diseases, and a method of preparation of the pharmaceutical composition.

### [BACKGROUND ART]

Cardiovascular diseases include heart-related diseases (myocardial infarction, high blood pressure, cardiac insufficiency, arrhythmia, arteriosclerosis) and vascular diseases (stroke, peripheral vascular diseases), and according to statistics of World Health Organization (WHO), despite remarkable developments in modern science and medicine, they have been reported as a disease having increased incidence and prevalence steadily over the past three decades, and it is one of fatal diseases in which the mortality rate due to cardiovascular system diseases accounts for the very high rate, which is about 30% of the total mortality rate, not only domestically but also globally. The increase of the incidence and mortality rate of cardiovascular diseases is mainly due to economic growth and westernization of lifestyle, and in addition, changes in dietary life such as high-fat diet, increased stress, aging of population due to life extension, lack of exercise, and the like. It is pointed out that a serious problem of cardiovascular diseases is that national excessive medical expenses and deterioration of life quality are caused by these diseases themselves and serious physical and social aftereffects after occurrence of these diseases. Accordingly, as the problem with the cardiovascular diseases are gradually importantly recognized, a lot of investment is being made in the research on pathogenesis as well as on prevention and treatment of these diseases globally, and therefore, as part of the research for development of a new antithrombotic agent with an effect of improving blood circulation, research for development of a new material showing an anticoagulant activity or a fibrinolytic activity has been actively conducted. However, as the prevalence and incidence of cardiovascular diseases have been steadily increasing worldwide, despite such research and development of drugs related to circulatory disorders so far, now there is an urgent need for development of an active substance and a food material which can inhibit and prevent cardiovascular outbreaks with development of a therapeutic for related diseases.

On the other hand, a thrombus is an aggregate of blood factors, and is mainly composed of aggregates of platelets and fibrin, and is usually formed to prevent excessive bleeding of blood vessels. When platelets come into contact with the surface of the subdermal layer, a reaction is initiated that creates a thrombus to block the vascular system, and this process is called hemostasis, and the process is very important in preventing excessive bleeding at the wound site. An arterial thrombus causes severe diseases due to local obstruction, while a venous thrombus mainly causes distant occlusion or embolism. Thus, medicines that inhibit platelet aggregation or do a thrombolytic action may be used for preventing and treating diseases related to thrombosis and embolism, such as myocardial infarction, angina, thrombophlebitis, arterial embolism, coronary and cerebral artery thrombosis, peripheral vascular or deep vein thrombosis, coronary thrombosis of arbitrary blood vessels, vein thrombosis, ischemic cerebral infarction, arteriosclerosis, high blood pressure, pulmonary hypertension, cerebral infarction, stroke, chronic arterial occlusion, pulmonary infarction, cerebral embolism, renal embolism, thromboembolism, and pathological symptoms after subarachnoid hemorrhage, percutaneous transluminal coronary angioplasty (PTCA) and thrombosis during stent insertion, restenosis after stent installation, catheter thrombotic occlusion or reocclusion, acute coronary artery syndrome, TIA (transient ischemic attack or acute cerebrovascular syndrome), cardiac insufficiency, chest pain caused by ischemic etiology, X syndrome and genuine diabetes. The mechanisms that block blood vessels include a blood coagulation mechanism and a thrombogenesis mechanism. The blood coagulation mechanism is that soluble fibrinogen is changed to poorly soluble fibrin and blood coagulates, and the main initiators include tissue factor (TF) which is an exogenous factor and factor VIIa which is an exogenous factor. At each stage, several coagulation factors are activated to form a fibrin colt (cross-linked fibrin molecule), which produces thrombin, and this substance is a strong platelet active material and stimulates the release of the activator. The thrombogenic mechanism is a spontaneous process to prevent bleeding from damaged blood vessels, rather than being formed in normal vascular endothelial cells, because platelets adhere to collagen and form viscous gelatin masses by activation with each other upon damaged vascular endothelium exposure.

Drugs that remove these thrombi include thrombolytics and antiplatelets. The thrombolytics are drugs that catalyze formation of serine protease plasmin from plasminogen and rapidly degrade thrombi, and include streptokinase, urokinase, APSAC, t-PA and the like, the antiplatelets regulated by Group 3 substances include drugs that are produced outside of platelets and interact with platelet membrane receptors, catecholamine, collagen, thrombin, and prostacyclin; drugs that are produced inside of platelets and interact with membrane receptors, ADP, PGD3, PGE2, and serotonin; and substances that are produced inside of platelets and act within platelets, prostaglandin endoperoxide, thromboxane A2 (TXA2), cAMP, cGMP, and Ca 2+. The antiplatelets also include A23187 which is drug that binds to the cell membrane as a calcium ionophore and causes aggregation of extracellular calcium into cells by producing a calcium passage, and U46619 which is a thromboxane A2 analog, which acts on a thromboxane receptor (TP) to show the same effect as thromboxane. However, the thrombolytics and antiplatelets developed to date have problems that cause various side effects such as hemostasis hyperinhibition, infertility, digestive disorders and the like, and therefore there is a great need for research on new bioactive compounds and drugs for antithrombosis with different mechanisms of action, increased efficacy and low toxicity.

Accordingly, the present inventors have confirmed that more excellent antithrombotic activity has shown when Oenanthe javanica extract and one or more kinds selected from the group consisting of Clopidogrel or its pharmaceutically acceptable salt and Cilostazol or its pharmaceutically acceptable salt as an antithrombotic agent are complexly administered, than the case in which each component is administered alone, thereby completing the present invention.

### [DISCLOSURE]

### [TECHNICAL PROBLEM]

Accordingly, an object of the present invention is to provide a pharmaceutical composition for antithrombosis comprising Oenanthe javanica extract, and one or more antithrombotic agents selected from the group consisting of Clopidogrel or its pharmaceutically acceptable salt and Cilostazol or its pharmaceutically acceptable salt, as active ingredients, and a method for inhibiting thrombi characterized by using the same.

Another object of the present invention is to provide a pharmaceutical composition for preventing, improving or treating thrombus-related diseases, comprising Oenanthe javanica extract, and one or more antithrombotic agents selected from the group consisting of Clopidogrel or its pharmaceutically acceptable salt and Cilostazol or its pharmaceutically acceptable salt, as active ingredients, and a method for preventing, improving or treating thrombus-related diseases characterized by using the same.

### [TECHNICAL SOLUTION]

As one aspect to achieve the above objects, the present invention relates to a pharmaceutical composition for antithrombosis comprising Oenanthe javanica extract, and one or more antithrombotic agents selected from the group consisting of Clopidogrel or its pharmaceutically acceptable salt and Cilostazol or its pharmaceutically acceptable salt, as active ingredients, and a method for inhibiting thrombi comprising administering the same, and a method for preparation thereof.

As another aspect, the present invention relates to a pharmaceutical composition for preventing or treating thrombus-related diseases comprising Oenanthe javanica extract, and one or more antithrombotic agents selected from the group consisting of Clopidogrel or its pharmaceutically acceptable salt and Cilostazol or its pharmaceutically acceptable salt, as active ingredients, and a method for preventing or treating thrombus-related diseases comprising administering the same.

Hereinafter, the present invention will be described in more detail.

The pharmaceutical composition for antithrombosis or for preventing or treating thrombus-related diseases according to the present invention comprises Oenanthe javanica extract, and one or more antithrombotic agents selected from the group consisting of Clopidogrel or its pharmaceutically acceptable salt and Cilostazol or its pharmaceutically acceptable salt, as active ingredients.

Water dropwort (Oenanthe javanica) is a perennial herb plant belonging to the family Apiaceae, and is aquatic or hygroscopic and the stem is long in the mud and white flowers bloom in July and August, and bears oval fruits. Inflorescences are opposite to leaves, split into 5 to 15 small peduncles, and each hangs 10 to 25 flowers. Branches split from the bottom of the stem, and spread to the side, and in the fall, roots descend from the node of the slender stalks to propagate. The stem is hairless and fragrant, and 20 to 50 cm high, and the leaves alternate, and 7 to 15 cm long, and are pinnate 1 to 2 times, and petioles are shorter as they go up. Small leaves are egg-shaped and pointed at the end, and have serrated edges. Among the water dropwort, wild water dropwort which grows in the field is called Oenanthe javanica, and it has no nutritional difference from general water dropwort, but has a slightly richer flavor.

Wild water dropwort includes all forms commonly used to obtain extract in the extract filed without any particular limit in its form. In addition, the extract may be solvent extract extracted with an extraction solvent, a fraction fractionized by adding a fraction solvent to extract prepared by extracting with an extraction solvent, or a purified product obtained by performing chromatography on the fraction. The extraction solvent may be water, sodium chloride aqueous solution, an organic solvent or a mixed solvent thereof which can be used in extraction of natural products, preferably, any one selected from the group consisting of water, sodium chloride aqueous solution, ethyl acetate, alcohols with 1 to 5 carbon atoms, and mixtures thereof. The organic solvent may be polar solvent such as linear or branched alcohols with 1 to 5 carbon atoms (C₁-C₅ linear or branched alcohol) such as methanol, ethanol, and the like, and non-polar solvent such as hexane or dichloromethane, and neutral solvent such as ketone with 3 to 5 carbon atoms and the like, or a mixed solvent thereof, and preferably, may be linear or branched alcoholic aqueous solution with 1 to 5 carbon atoms such as 30 to 90 (v/v)% ethanol, more preferably, 50 to 80 (v/v)% ethanol aqueous solution. The fraction solvent may be linear or branched alcohol with 1 to 5 carbon atoms, ethyl acetate or a mixture thereof. In one preferable aspect, the Oenanthe javanica extract of the present invention may be an ethyl acetate fraction of the crude extract extracted from Oenanthe javanica with ethanol. The Oenanthe javanica extract may be prepared by common preparation methods of plant extract. More specifically, it may be performed by the method of adding an extraction solvent to Oenanthe javanica of which impurities are removed and conducting an extraction process. The extraction process may be enfleurage extraction, maceration extraction, pressurized extraction, reflux extraction or ultrasonic extraction. The preparation of the fraction of Oenanthe javanica may be performed by a method of obtaining fractions depending on the polarity of the fraction solvent after adding a fraction solvent to the extract extracted by the extraction method,that is the crude extract or crude extracted solution. The method of obtaining fractions may be conducting by separation or fractionation by layer separation. The chromatography for purification of the fractions may be performed by various chromatography such as silica gel column chromatography, thin layer chromatography (TLC) or high-performance liquid chromatography (HPLC), and the like. In addition, the extract may be concentrated or the solvent may be removed by performing a decompression filtration process or additionally performing concentration and/or lyophilization, after conducting the extraction, fractionation or purification process. Accordingly, the Oenanthe javanica extract of the present invention is used as the meaning including a dried product of dried extract by a common method, a concentrate of concentrated extract by a common method, and dilution of the extract, dried product or concentrate.

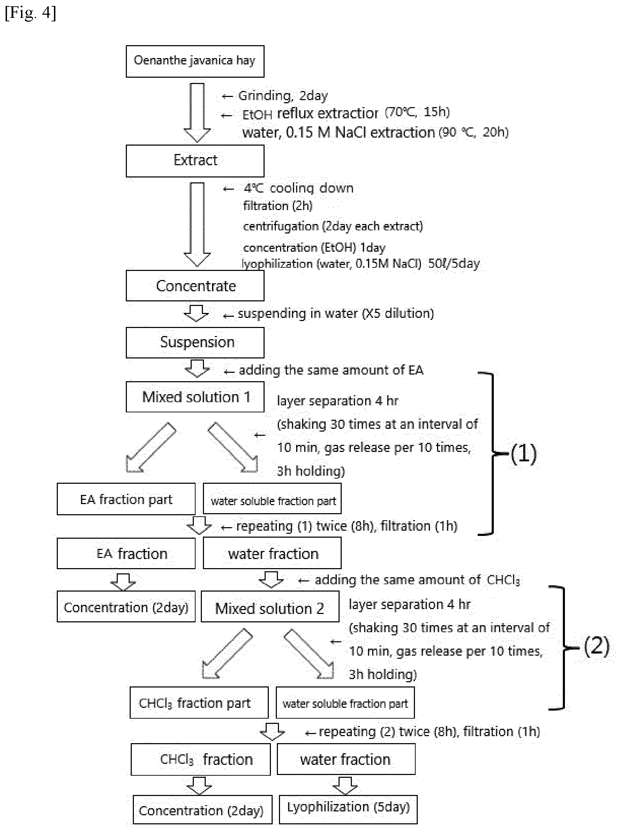

Specifically, in one example of the present invention, 5kg Oenanthe javanica hay was washed and shredded, and 35L spirit (95(v/v)%) ethanol aqueous solution was added to primarily extract at 70°C for 5 years, and after the primary extraction, secondary and tertiary extraction were conducted using 15L spirit for 5 hours under the same condition as the primary extraction. After completing the extraction, it was collected and was refrigerated at 4°C. The extract was sieved with 100 mesh, and then was filtered with 10*µ*m filter paper, and this was refrigerated at 4°C again, and this was concentrated (5ℓ/1hr) to prepare the Oenanthe javanica extract according to the present invention. The flow chart of one preparative example of this specific Oenanthe javanica extract was shown in FIG. 4.

In addition, the composition according to the present invention comprises one or more kinds selected from the group consisting of Clopidogrel or its pharmaceutically acceptable salt and Cilostazol or its pharmaceutically acceptable salt as an antithrombotic agent, as another active ingredient.

Among then, Clopidogrel has the chemical name of methyl(+)-(S)-α-(o-chlorophenyl)-6,7-dihydrothieno[3,2-c]pyridine-5(4H)-acetate (dextrorotatory enantiomer), which prevents ischemic events caused by vascular diseases such as atherosclerosis and heart attack by having a platelet inhibitory activity. Clopidogrel has an antithrombotic effect by inhibiting platelet aggregation, and thus it is useful for prevention and treatment of various kinds of vascular diseases such as arteriosclerosis, myocardial infarction, and peripheral arterial occlusion. As salts of Clopidogrel, hydrochloride, hydrogen sulfate, bromate, taurochlorate, and the like have been known, and currently, in clinical practice, it is used in the form of Clopidogrel hydrogen sulfate (or called sulfate, C₁₆H₁₆ClNO₂S·H₂SO₄). Accordingly, preferably, the pharmaceutically acceptable salt of Clopidogrel which can be comprised in the composition according to the present invention may be hydrogen sulfate.

Furthermore, Cilostazol is 6-[4-(1-cyclohexyl-1H-tetrazol-5-yl)-butoxy]-3,4-dihydrocarbostyryl, and shows a phosphodiesterase inhibitory action, an antitumor action, antihypertensive action, and an anti-inflammatory action, as well as a high platelet aggregation inhibitory action, and therefore it is a widely used drug as not only a phosphodiesterase inhibitor but also an antithrombotic agent, a cerebral circulation enhancer, an anti-inflammatory, an antitumor agent, an antihypertensive agent, and an anti-asthmatic agent. The pharmacologically acceptable salt of Cilostazol may be easily prepared by reacting Cilostazol with a pharmacologically acceptable acid. The pharmacologically acceptable acid includes for example, inorganic acids such as chloride, sulfate, phosphate, and hydrobromic acid, and organic acids such as oxalate, maleic acid, fumaric acid, malic acid, tartaric acid, citric acid and benzoate.

The Oenanthe javanica extract, and one or more kinds selected from the group consisting of Clopidogrel or its pharmaceutically acceptable salt and Cilostazol or its pharmaceutically acceptable salt as an antithrombotic agent, as active ingredients in the composition according to the present invention may be appropriately adjusted by use type and purpose, patient condition, type of symptoms and severity and the like, and it may be decreased or increased according to needs of users, and it may be suitably decreased or increased according to various factors such as diet, nutritional status, and progression of thrombi, and the like. In addition, the weight ratio of the Oenanthe javanica extract, and one or more kinds selected from the group consisting of Clopidogrel or its pharmaceutically acceptable salt and Cilostazol or its pharmaceutically acceptable salt that is an antithrombotic agent in the composition according to the present invention may be 200:1 to 1:200, preferably, 100:1 to 1:100, and more preferably 1:1 to 1:100.

The composition according to the present invention may be administered into a mammal including humans by various routes. The administration method may be any of commonly used methods, and for example, it may be administered orally or parenterally (for example, skin, intravenous, intramuscular, subcutaneous) or the like, and preferably, it may be administered orally. The composition of the present invention may be used by formulating to oral formulations such as powders, granules, tablets, capsules, liquids, suspension, emulsion, syrup, aerosol and the like, or parenteral formulations such as percutaneous agents, suppositories, ointment and sterile injectable solution, by common methods, respectively.

The composition of the present invention may further contain an adjuvant such as a carrier, an excipient and a diluent that is pharmaceutically suitable and physiologically acceptable, in addition to the mixed extract. For example, preparations for oral administration may be formulated using an excipient, a binding agent, a disintegrating agent, a glydent, a solubilizing agent, a suspending agent, a preservative or an extender, or the like.

The dose of the pharmaceutical composition of the present invention may be determined by a specialist according to various factors such as patient condition, age, body weight, degree of bronchial or lung damage due to asthma, degree of disease progression and the like. In addition, it is intended to contain a daily dose or its 1/2, 1/3 or 1/4 dose of the pharmaceutical composition per unit dose, and it may be administered 1 to 6 times a day.

Accordingly, as other aspect, the present invention relates to a method for inhibiting thrombi comprising administering the pharmaceutical composition. The matters relating to the pharmaceutical composition may be applied for matters of administration methods and dosages and the like.

The composition of the present invention may be usefully used as a composition for antithrombosis and a pharmaceutical composition for prevention or treatment of thrombus diseases, as it shows an excellent anti-thrombotic effect by effectively inhibiting aggregation of platelets. As other aspect, the present invention relates to a method for prevention or treatment of thrombus diseases comprising administering the pharmaceutical composition. The matters relating to the pharmaceutical composition may be applied for matters of administration methods and dosages and the like.

The thrombus disease includes one or more kinds selected from the group consisting of myocardial infarction, angina, thrombophlebitis, arterial embolism, coronary thrombosis, cerebral artery thrombosis, peripheral vascular thrombosis, deep vein thrombosis, coronary thrombosis, vein thrombosis, ischemic cerebral infarction, arteriosclerosis, high blood pressure, pulmonary hypertension, cerebral infarction, stroke, chronic arterial occlusion, pulmonary infarction, cerebral embolism, renal embolism, thromboembolism, pathological symptoms after subarachnoid hemorrhage, percutaneous transluminal coronary angioplasty (PTCA) or thrombosis during stent insertion, restenosis after stent installation, catheter thrombotic occlusion or reocclusion, acute coronary artery syndrome, TIA (transient ischemic attack or acute cerebrovascular syndrome) and cardiac insufficiency, but not limited thereto.

As other aspect, the present invention relates to a method of preparation of the pharmaceutical composition for antithrombosis or for preventing or treating thrombus-related diseases comprising Oenanthe javanica extract and Clopidogrel or its pharmaceutically acceptable salt. Specifically, the present invention relates to a method for preparation of the composition for antithrombosis or for preventing or treating thrombus-related diseases, comprising
a) preparing Oenanthe javanica extract;
b) mixing the Oenanthe javanica extract prepared in the step a); and one or more kinds selected from the group consisting of Clopidogrel or its pharmaceutically acceptable salt and Cilostazol or its pharmaceutically acceptable salt that is an antithrombotic agent, and a pharmaceutically acceptable carrier and/or an additive; and
c) preparing a preparation by formulating a mixture prepared in the step b).

Preferably, in the method of preparation of the present invention, in the step a), Oenanthe javanica extract may be prepared by using various extraction solvents and extraction methods aforementioned relating to the composition. In addition, as the step b), after mixing components of the preparation, for increase of fluidity, secureness of content uniformity of the preparation, and the like, additionally, it may be pelletized or granulated to a suitable size through a process such as sieving and the like. Then, the pellets or granules of the mixtures prepared as above may be further mixed with a pharmaceutically acceptable carrier and/or an additive before further formulation.

In the step c), for the method for formulating the mixture prepared in the step b), any known method used for preparing preparations may be used, but commonly, in case of tablets, it may be prepared by the method of tableting by adding a uniform amount of mixture to a tableting machine, and direct compression molding (direct compression), wet granulation or dry granulation, which is commonly used in preparation of tablets, may be used without limitation. Furthermore, in case of capsules, it may be prepared by uniformly adding the mixture of the step b) in a desired content into an empty capsule such as a gelatin capsule in an appropriate size.

### [ADVANTAGEOUS EFFECTS]

As described above, the pharmaceutical composition of the present invention comprises Oenanthe javanica extract, and one or more kinds selected from the group consisting of Clopidogrel or its pharmaceutically acceptable salt and Cilostazol or its pharmaceutically acceptable salt as an antithrombotic agent, as active ingredients together, and therefore it exhibits an excellently synergistic effect of inhibiting thrombosis compared to combinations of other antithrombotic agents, thereby being usefully used as an antithrombotic agent and a prophylactic or therapeutic agent of thrombus diseases in the pharmaceutical industry.

### [BRIEF DESCRIPITON OF THE DRAWINGS]

FIG. 1 is a graph showing the time to stop blood flow through carotid artery by thrombus formation when Oenanthe javanica extract according to the present invention and Clopidogrel are co-administered (Time to occlusion, TTO). In the FIG. 1, Clo 1 means Clopidogrel 1mg/kg, and Clo 2 means Clopidogrel 2mg/kg.
FIG. 2 is a graph of measuring the thrombus weight when Oenanthe javanica extract according to the present invention and Clopidogrel are co-administered. In the FIG. 2, Clo 1 means Clopidogrel 1mg/kg, and Clo 2 means Clopidogrel 2mg/kg.
FIG. 3 is a graph of confirming the platelet aggregation inhibitory activity according to combinations of Oenanthe javanica extract according to the present invention extracted by various extraction solvents and various antithrombotic agents.
FIG. 4 is a flow chart illustrating one preparative example of Oenanthe javanica extract according to the present invention.

### [DETAILED DESCRIPTION OF THE EMBODIMENTS]

Hereinafter, the present invention will be specifically described by examples and experimental examples, but the following examples and experimental examples are intended to illustrate the present invention only, and the scope of the present invention is not to be construed as being limited to the following examples and experimental examples.

### 1. Preparative example 1: Preparation of Oenanthe javanica water extract, NaCl extract and ethanol extract

In order to prepare the Oenanthe javanica extract according to the present invention, Oenanthe javanica water extract, Oenanthe javanica NaCl extract and ethanol extract were prepared by the following method.

First, in case of water and 0.15M NaCl extract, Oenanthe javanica hay 5 kg was primarily extracted at 90°C for 17 hours, using 35L of water and 0.15M NaCl solution, respectively. Furthermore, after the primary extraction, the secondary extraction was conducted for 3 hours using 15L of the same extraction solvent under the same condition. The extracted extract was collected and was refrigerated at 4°C, and then it was sieved with a 100mesh sieve and was filtered with a 10*µ*m filter. After that, the filtered filtrate was centrifuged (4,500rpm 30 min), and then was filtered with filter paper, and it was refrigerated at 4°C and was lyophilized (50ℓ/5day).

In case of ethanol extract, Oenanthe javanica hay 5kg was washed and shredded, and 35L of spirit (95(v/v)%) ethanol aqueous solution was added and it was primarily extracted at 70°C for 5 hours, and after the primary extraction, the secondary and tertiary extraction were conducted using 15L spirit for 5 hours under the same condition as the primary extraction. After completing the extraction, it was collected and was refrigerated at 4°C. The extraction was sieved with a 100mesh sieve and was filtered with a 10*µ*m filter, and this was refrigerated at 4°C again, and it was concentrated (5ℓ/1hr) to prepare the Oenanthe javanica extract according to the present invention. The specific flow chart of one preparative example of the Oenanthe j avanica extract was shown in FIG. 4.

### 2. Preparative example 2: Preparation of fractions of Oenanthe iavanica extract

Various solvent fractions of the Oenanthe javanica extract were prepared using the Oenanthe javanica water extract, NaCl extract, and ethanol extract prepared in Preparative example 1. Specifically, the concentrates of the Oenanthe javanica extract prepared in Preparative example 1 was suspended in water, and chloroform in the same amount as the suspension was added and mixed, and then layers were separated for about 4 hours, and this process was repeated two more times. After adding and mixing the same amount of ethyl acetate to the separated water fractions, layers were separated for about 4 hours, and this process was repeated two more times. In addition, the same amount of butanol was added and mixed to the separated water fractions, and then layers were separated for about 4 hours, and this process was repeated two more times. Then, each of separated fractions was concentrated (5ℓ/h) to prepare each solvent fraction, and its flow chart was shown in FIG. 4.

### 3. Experimental example 1: Evaluation of antithrombotic effect of Oenanthe iavanica extract using rat arterial thrombosis model

In order to confirm the antithrombotic efficacy in case of single administration and co-administration of the Oenanthe javanica extract which is one of the active ingredients of the composition according to the present invention, and 4 kinds of known antithrombotic agents, an animal experiment was progressed using FeCl₃-induced carotid arterial thrombosis rat model.

### 3-1. Experimental reagents and samples

As the Oenanthe extract according to the present invention, the ethyl acetate fractions of the Oenanthe javanica ethanol extract prepared in Preparative example 2 were used, and Clopidogrel, Cilostazol, Ginko biloba extract and the like were prepared in-house and used. FeCl₃, Arabic gum and Aspirin were purchased from Sigma-Aldrich (Yongin, Korea). All samples were suspended in 5% Arabic gum to progress the animal experiment.

### 3-2. Experimental animals and experimental groups

SD rat (male, 6 weeks old) was purchased from Orientbio company (Seoul, Korea), and was adapted in Konkuk University RIC center animal laboratory for over 1 week, and then was fasted for 18 hours at minimum, and then was used in the experiment.

The experimental groups were divided into 8 groups in total as follows, and 7 rats were organized per group for accurate efficacy evaluation due to the nature of the disease model.

### 1) Single administration test (5 experimental groups):

Control (1 group): 5% Arabic gum (solvent administration group)
Drug group (2 groups): Clopidogrel 1, 2 mg/kg (positive control material)
Oenanthe javanica extract according to the present invention (2 groups): Oenanthe javanica extract 50 and 100 mg/kg

### 2) Complex administration test (3 experimental groups):

Oenanthe javanica extract 50 mg/kg + Clopidogrel 1 mg/kg (2 set)
Oenanthe javanica extract 100 mg/kg + Clopidogrel 1 mg/kg

### 3-3. Experimental method

The carotid arterial thrombosis animal (rat) model was prepared in reference to methods by JH Cho et al. (Thrombosis Research 100: 97-107, 2000), JH Cho et al. (Thromb Haemost 86: 1512-1520, 2001), and KD Kurz et al. (Thrombosis Research 60:269-280, 1990), and the time to stop carotid artery flow due to FeCl₃-induced thrombosis (TTO, time to occlusion) and the weight of thrombi produced in the carotid artery in the thrombosis animal model by the following specific method.
1) Orally administrate a test substance, solvent, a positive control material, and the like to a rat in a certain amount to weight.
2) Administrate an anesthetic according to the weight of the rat in about 40 minutes after the oral administration (intraperitoneal injection of pentobarbital Na 60 mg/kg).
3) Fix the rat on a lab table on a heating pad with paper tape and cut near the neck where the shoulder line leads (keep the body temperature of the rat to 37±0.5°C using a heat generating lamp during the experiment).
4) Conduct tracheotomy (Secure the respiratory tract).
5) Cut with scissors in small, and then carefully expose the left carotid artery, using two forceps to rip off muscles.
6) Remove the adipose tissue attached to the blood vessel and further cut muscles surrounding the both ends of the blood vessel and insert a small tweezer under the blood vessel to fix it (prevent the blood vessel from squeezing even with the tweezer).
7) Place parafilm (1×3 cm) under the blood vessel of the left carotid artery (role of fixing a flow probe).
8) After washing the left carotid artery with 0.9% saline, a transonic flow probe was inserted into the blood vessel.
9) Insert the transonic flow probe to the carotid artery, and then connect with a transonic flow measuring instrument to confirm that the blood flow is properly monitored.
10) Between where to place FeCl_{3,} and the probe, roll filter paper and place it on the blood vessel.
11) Soak filter paper (Whatman No.1) disc (1×2 mm) to 20% FeCl₃ (in 0.9% NaCl) solution.
12) In 1 hour after orally administering the sample, attach a filter paper disc to the top part of the blood vessel in the downward direction of the probe, and remove the filter in 10 minutes from that time.
13) Consistently monitor the carotid patency of the carotid artery, while measuring the carotid artery blood flow by a flowmeter for 60 minutes after attaching FeCl₃ to the blood vessel.
14) After the experiment, tie and separate a piece of the blood vessel of sufficient length near the attached filter paper disc with operation thread using a blood vessel clamp to measure the weight of thrombi produced in the blood vessel.

### 3-4. Statistical analysis

All results were shown by Mean±SEM, and were analyzed at the p<0.05 level of the significant difference by One-way ANOVA and Dunnett's Multiple Comparison Test and Student's unpaired t test (one-tailed).

### 3-5. Result

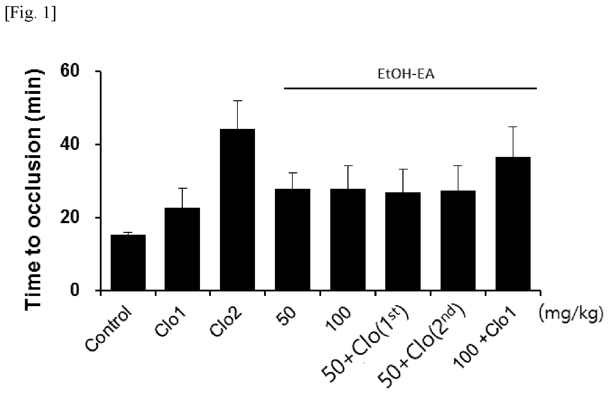

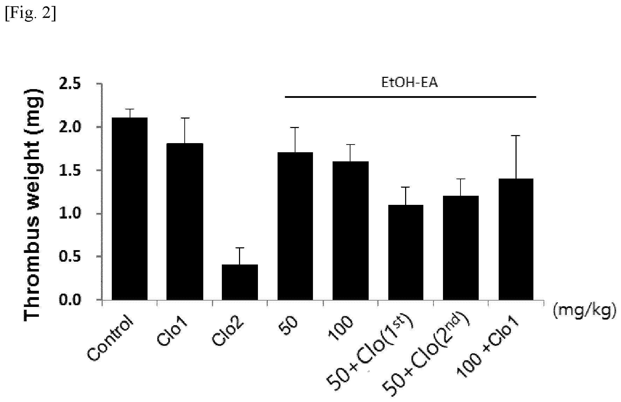

The effect to 2 parameters relating to thrombosis (time to occlusion, TTO, min; thrombus weight, mg), in case of single administration or co-administration of the sample and positive control material was shown in the following Table 1 and FIG. 1 and FIG. 2 using the rat carotid artery thrombosis model.

### 1) Thrombosis induction by iron chloride solution

It was observed that the time to stop the blood flow through the carotid artery by thrombogenesis (time to occlusion, TTO, min) was 15.1 minutes and the weight of thrombi formed in the carotid artery at the site where the iron chloride stimulus was applied was 2.1 mg, when the carotid artery was exposed to the filter paper piece (1x2 mm) saturated with iron chloride (20%) in the rat anesthetized with pentobarbital, and therefore it could be seen that thrombi were induced well in the carotid artery due to hemangioendothelial damages by iron chloride.

### 2) Effect of single administration of Oenanthe iavanica extract according to the present invention and positive control material

Clopidogrel used as the positive control material showed the tendency to increase TTO and reduce the weight of thrombi dose-dependently compared to the solvent administration group, when orally administering it at 1 or 2 mg/kg dose (high dose administration group : p<0.05 versus solvent administration group; See FIG. 1 and FIG. 2, and Table 1 as follows).

The Oenanthe javanica extract according to the present invention showed the tendency to increase TTO (low and high dose administration groups: p<0.05 versus solvent administration group) and reduce the weight of thrombi (high dose administration group: p<0.05 versus solvent administration group. See FIG. 1 and FIG. 2, and Table 1), compared to the solvent administration group, when orally administering it at 50 and 100 mg/kg doses.

**[Table 1]**

| | **Contro l group** | **Clopi dogr el 1mg/ kg** | **Clopid ogrel 2mg/k g** | **Oenan the javanic a EtOH-EA fractio 50mg/ kg** | **Oenanth e javanica EtOH-EA fraction 100mg/k g** | **Oenanthe javanica EtOH-EA fraction 50mg/kg+ Clopidogrel 1mg/kg (1** 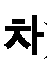 **)** | **Oenanthe javanica EtOH-EA fraction 50mg/kg+ Clopidogrel 1mg/kg (2** 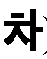 **)** | **Oenanthe javanica EtOH-EA fraction 100mg/kg+ Clopidogrel 1mg/kg** |
|---|---|---|---|---|---|---|---|---|
| **TTO** | 66.8% | 100% | 195.1% | 123.5% | 123.0% | 118.1% | 121.2% | 160.6% |
| **Weig ht of thro mbi** | 116.7% | 100% | 22.2% | 94.4% | 88.9% | 61.1% | 66.7% | 77.8% |

### 3) Effect of co-administration of Oenanthe javanica extract according to the present invention and Clopidogrel

### Action on TTO

When co-administering each dose of the Oenanthe javanica extract according to the present invention (50, 100 mg/kg) with Clopidogrel 1 mg/kg dose at the same time, co-administration with Oenanthe javanica extract 50 mg/kg showed a similar level of TTO to single administration of Oenanthe javanica extract, and longer TTO than Clopidogrel single administration. On the other hand, co-administration with Oenanthe javanica extract 100 mg/kg tended to show longer TTO than single administration of Oenanthe javanica extract or Clopidogrel single administration (See FIG. 1 and FIG. 2, and Table 1).

### Action on carotid artery thrombus weight

When co-administering each dose of the Oenanthe javanica extract according to the present invention (50, 100 mg/kg) with Clopidogrel 1 mg/kg dose at the same time, co-administration with Oenanthe javanica extract 50 mg/kg showed the effect of decreasing the weight of thrombi compared to single administration of Oenanthe javanica extract and Clopidogrel single administration (See FIG. 1 and FIG. 2, and Table 1). Co-administration with Oenanthe javanica extract 100 mg/kg showed the tendency to decrease the weight of thrombi than single administration of Oenanthe javanica extract or Clopidogrel single administration.

### 4. Experimental example 2: Evaluation of platelet aggregation inhibitory activity by extract

With the light transmission of PRP and WP as 0% and the light transmission of platelet poor plasma (PPP), suspension buffer as 100%, the light transmission according to the degree of aggregation of platelets was measured. As the measurement method, the method disclosed in Watson et al ("Inhibition of mitogen-activated protein kinase kinase does not impair primary activation of human platelets," Biochem J. 1996 Aug 15; 318(Pt 1): 207212) was used.

Specifically, it was performed according to the following evaluation method, and concentrated platelets used then were diluted to 1/7 and were used at 37°C within 2 hours, and ethyl acetate fractions of Oenanthe javanica ethanol extract, ethyl acetate fractions of Oenanthe javanica water extract, ethyl acetate fractions of Oenanthe javanica 0.15 NaCl extract, Clopidogrel, Cilostazol, and Aspirin were used as samples, respectively.

### Evaluation method

1. Set a four-channel aggregometer of Chrono-Log company (preheating 30 minutes, 37°C)
2. Add stir bars to plastic cuvettes in PRP 4 channels and add the platelet diluted solution (483*µ*ℓ) and increase the temperature (37°C). Add the plastic cuvette and suspension buffer 500*µ*ℓ in PPP compartment
3. Operate the aggregometer and set the baseline
   - Prepare the standard, Ascorbic acid 1mg with DMSO 1ml
4. Add a sample and a coagulant
   - after adding the control to No. 1 channel and the sample to No. 2,3,4, react for about 1 minute
   - Treat the coagulant and measure for 5 minutes
5. Store results
   - Designate the aggregation start and end points by selecting "Set Start and Stop Time"
   - Confirm the slop and amplitude by selecting "Compute slop and amplitude"

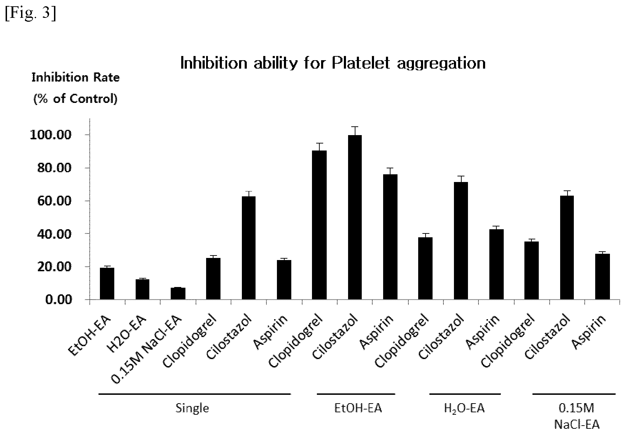

The result for the test of confirming the platelet aggregation inhibitory activity was shown in the following Table 3. As could be seen in FIG. 3, it could be seen that the ethyl acetate extract of the ethanol extract among fractions of the Oenanthe j avanica extract showed the most excellent platelet aggregation inhibitory activity in case of single administration, even as well as co-administration with Clopidogrel, Cilostazol or Aspirin.

## Claims

1. A composition for antithrombosis comprising Oenanthe javanica extract; and one or more antithrombotic agents selected from the group consisting of Clopidogrel or its pharmaceutically acceptable salt and Cilostazol or its pharmaceutically acceptable salt as active ingredients.

2. The composition of antithrombosis according to claim 1, wherein the Oenanthe javanica extract is water, sodium chloride aqueous solution, organic solvent or their mixed solvent extract of Oenanthe javanica.

3. The composition of antithrombosis according to claim 2, wherein the organic solvent is ethanol.

4. The composition of antithrombosis according to claim 1, wherein the Oenanthe javanica extract is fractions of water, sodium chloride aqueous solution, organic solvent or their mixed solvent extract of Oenanthe javanica.

5. The composition of antithrombosis according to claim 4, wherein the Oenanthe javanica extract is ethyl acetate fractions of Oenanthe javanica ethanol extract.

6. The composition of antithrombosis according to claim 1, wherein the pharmaceutically acceptable salt of Clopidogrel is hydrogen sulfate.

7. The composition of antithrombosis according to claim 1, wherein the weight ratio of the Oenanthe javanica extract to the one or more selected from the group consisting of Clopidogrel or its pharmaceutically acceptable salt and Cilostazol or its pharmaceutically acceptable salt (Oenanthe javanica extract: one or more kinds selected from the group consisting of Clopidogrel or its pharmaceutically acceptable salt and Cilostazol or its pharmaceutically acceptable salt) is 200 : 1 to 1 : 200.

8. A composition for preventing or treating thrombus-related diseases comprising Oenanthe javanica extract; and one or more kinds of antithrombotic agents selected from the group consisting of Clopidogrel or its pharmaceutically acceptable salt and Cilostazol or its pharmaceutically acceptable salt as active ingredients.

9. The composition for preventing or treating thrombus-related diseases according to claim 8, wherein the thrombus-related disease is one or more kinds selected from the group consisting of myocardial infarction, angina, thrombophlebitis, arterial embolism, coronary thrombosis, cerebral artery thrombosis, peripheral vascular thrombosis, deep vein thrombosis, coronary thrombosis, vein thrombosis, ischemic cerebral infarction, arteriosclerosis, high blood pressure, pulmonary hypertension, cerebral infarction, stroke, chronic arterial occlusion, pulmonary infarction, cerebral embolism, renal embolism, thromboembolism, pathological symptoms after subarachnoid hemorrhage, percutaneous transluminal coronary angioplasty (PTCA) or thrombosis during stent insertion, restenosis after stent installation, catheter thrombotic occlusion or reocclusion, acute coronary artery syndrome, TIA (transient ischemic attack or acute cerebrovascular syndrome) and cardiac insufficiency.

10. A method for inhibiting thrombi, comprising administering the composition for antithrombosis according to claim 1.

11. A method for prevention or treatment of thrombus-related diseases, comprising administering the composition for preventing or treating thrombus-related diseases according claim 8.

12. A method for preparation of the composition according to any one of claims 1 to 9, comprising
a) preparing Oenanthe javanica extract;
b) mixing the Oenanthe javanica extract prepared in the step a); and one or more kinds of antithrombotic agents selected from the group consisting of Clopidogrel or its pharmaceutically acceptable salt and Cilostazol or its pharmaceutically acceptable salt, and a pharmaceutically acceptable carrier and/or an additive; and
c) preparing a preparation by formulating a mixture prepared in the step b).
